(19) **Europäisches Patentamt / European Patent Office / Office européen des brevets**

(11) **EP 1 945 163 B1**

(12) # EUROPEAN PATENT SPECIFICATION

(45) Date of publication and mention
of the grant of the patent:
**20.04.2016 Bulletin 2016/16**

(51) Int Cl.:
*A61F 13/15* (2006.01)   *D01D 5/08* (2006.01)
*D01F 6/06* (2006.01)

(21) Application number: **06809643.7**

(86) International application number:
**PCT/IB2006/053851**

(22) Date of filing: **18.10.2006**

(87) International publication number:
**WO 2007/046069 (26.04.2007 Gazette 2007/17)**

(54) **ABSORBENT ARTICLE COMPRISING AUXETIC MATERIALS**

SAUGFÄHIGER ARTIKEL AUS AUXETISCHEN MATERIALIEN

ARTICLE ABSORBANT COMPRENANT DES MATERIAUX AUXETIQUES

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **21.10.2005 US 728890 P**

(43) Date of publication of application:
**23.07.2008 Bulletin 2008/30**

(73) Proprietor: **The Procter & Gamble Company
Cincinnati, OH 45202 (US)**

(72) Inventors:
• **ROE, Donald, Carroll
West Chester, Ohio 45069 (US)**
• **SCHMIDT, Mattias
65510 Idstein (DE)**

(74) Representative: **L'Huillier, Florent Charles et al
Procter & Gamble Service GmbH
Frankfurter Strasse 145
61476 Kronberg/Taunus (DE)**

(56) References cited:
**WO-A-00/53830    WO-A-95/13775**

• **BURKE M: "A STRETCH OF THE IMAGINATION"
NEW SCIENTIST, REED BUSINESS
INFORMATION, SURREY, GB, vol. 154, no. 2085,
7 June 1997 (1997-06-07), pages 36-39,
XP002107878 ISSN: 0262-4079**
• **STOTT P J ET AL: "A GROWTH INDUSTRY"
MATERIALS WORLD, THE INSTITUTE OF
MATERIALS, LONDON, GB, vol. 8, October 2000
(2000-10), pages 12-14, XP008024964 ISSN:
0967-8638**

Printed by Jouve, 75001 PARIS (FR)

**Description**

FIELD OF THE INVENTION

This invention relates to the materials comprising a wearable absorbent article. In particular this invention relates to the use of auxetic materials in the construction of such absorbent articles.

BACKGROUND OF THE INVENTION

Users, for example caregivers of infants, rely on disposable absorbent articles to make their lives easier. Disposable absorbent articles, such as adult incontinence articles and diapers, are generally manufactured by combining several components. These components typically include a liquid-permeable topsheet; a liquid-impermeable backsheet attached to the topsheet; and an absorbent core located between the topsheet and the backsheet. When the disposable article is worn, the liquid-permeable topsheet is positioned next to the body of the wearer. The topsheet allows passage of bodily fluids into the absorbent core. The liquid-impermeable backsheet helps prevent leakage of fluids held in the absorbent core. The absorbent core generally is designed to have desirable physical properties, e.g. a high absorbent capacity and high absorption rate, so that bodily fluids can be transported from the skin of the wearer into the disposable absorbent article.

While contemporary absorbent articles provide excellent leakage resistance and wearer comfort there are still areas for improvement. Nonlimiting examples include:

- During application of pants (refastenable or permanently seamed) it is necessary to stretch the waist region in order to accommodate the larger circumference of a wearer's hips (compared to waist circumference). However application of tension can cause the waist opening to assume a flattened oval configuration or even fully collapse making it difficult to insert the wearer's legs into the opening and to pull the pant over a wearer's buttocks. Thus, it would be desirable for the waist region to be stiffer during application so as to resist collapse of the waist opening so as to maintain an open geometry. However, simply stiffening the waist region is undesirable because of reduced wearer comfort after application. Thus, it would be desirable to provide pant-type absorbent article with stiffness while it is being applied that reverts to a soft flexible configuration after application.

- Leakage control via cuffs on an absorbent article requires balancing contractive force with wearer comfort. As is readily recognized, maximizing contractive force would minimize leakage around a cuff. However, such maximization has negative wearer comfort consequences. It is well known that if contractive force is too high there is resulting pressure marking on a wearer's skin (often called "redmarking"). Thus, it would be desirable for a cuff material to be able to maintain contact with a wearer's skin with little or no redmarking even as the stress on the cuff increases due to the added weight of absorbed exudates.

- Failure of fastening systems can lead to catastrophic failure of the protective function of an absorbent article in certain types of fastening systems (e.g., the tab and slot systems described in US Pat. 6,432,098 and in published US Pat. Appl. 2003/0233082 A1. Such failure can, for example, be due to the flexibility of one, or both, of the components of the closure system having a high enough flexibility that they become disengaged as a result of a tension spike due to normal wearer motion. Similarly, in hook and loop fastening systems (e.g., those described in US Pats. 3,083,737, 5,019,065 and 5,058,247), high tension can cause the system to release due to loop separation from the hooks due to flexibility of one of the hooks or the loops. Thus it would be desirable for a closure system to comprise a material that is thin and flexible while under low tension but to increase in stiffness as tension increases.

- Typical materials used for topsheets and backsheets neck down when under tension. Thus, as the weight of an absorbent article increases due to application of the absorbent article or due to absorption of exudates, there is a risk of reduced body coverage due to stretching and necking of such components. Thus, it would be desirable to fabricate an absorbent article from components that are resistant to necking when under tension.

- As core components acquire exudates the rate of acquisition for such exudates decreases. In some instances this is due to void volume becoming filled with a "gush" of exudate and not being fully drained by other core components prior to a subsequent "gush". Thus it would be desirable to provide materials for use as a core component that has improved an acquisition rate for a first "gush" and for subsequent "gushes" while, at the same time, having a desirably compact volume prior to application of the absorbent article to a wearer.

Auxetic materials have properties that are suitable for addressing many, if not all of these needs. For example, a waist band comprising an auxetic foam would expand in thickness as it is being stretched providing increased resistance to collapse of the waist opening while the waist band is under tension during application of the absorbent article while being soft and flexible after tension is released. Similarly, an auxetic nonwoven material would have a desirably low volume when not under tension while, at the same time, an increased volume (e.g., for temporary storage of exudates) when tension is applied.

Auxetic materials are described in US Pats. 4,688,557 and 6,878,320. A process for producing auxetic foams is described in PCT Published Application WO 99/255530. Nonwoven structures, especially cellulosic tissue, are described in US Published Application 2005/014233A1. However, the art has failed to recognize the broad utility of auxetic materials as a response to the continuing need to improve the comfort and functionality of disposable absorbent articles.

SUMMARY OF THE INVENTION

The present invention is a disposable absorbent article having a longitudinal centerline and a lateral centerline, a front waist region with a front waist edge, a rear waist region with a rear waist edge, a crotch region disposed between said front and rear waist regions and two spaced apart longitudinal side edges which join the front waist edge to the rear waist edge. The absorbent article is assembled from a collection of components where at least a portion of at least one of the waist feature component exhibits an auxetic response to an applied stress.

In some embodiments of the invention the auxetic response has a Poisson ratio of between about -0.01 and -1.0.

BRIEF DESCRIPTION OF THE DRAWINGS

FIG. 1 is a plan view of an exemplary diaper in a flat, uncontracted state.

FIG. 2 is a diagrammatic representation of the behavior under tension of a non-auxetic material.

FIG. 3 is a diagrammatic representation of the behavior under tension of an auxetic material.

FIGs. 4a and 4b are a diagrammatic representation of the behavior of an auxetic fabric when relaxed and when under tension.

FIG. 5 is a diagrammatic representation of an auxetic structure comprising an assembly of macro elements.

DETAILED DESCRIPTION OF THE INVENTION

As used herein, the following terms shall have the meaning specified thereafter:

"Disposable," in reference to absorbent articles, means that the absorbent articles are generally not intended to be laundered or otherwise restored or reused as absorbent articles (*i.e.,* they are intended to be discarded after a single use and, preferably, to be recycled, composted or otherwise discarded in an environmentally compatible manner).

"Absorbent article" refers to devices which absorb and contain body exudates and, more specifically, refers to devices which are placed against or in proximity to the body of the wearer to absorb and contain the various exudates discharged from the body. Exemplary absorbent articles include diapers, training pants, pull-on pant-type diapers (*i.e.,* a diaper having a preformed waist opening and leg openings such as illustrated in US Pat. No. 6,120,487), refastenable diapers or pant-type diapers, incontinence briefs and undergarments, diaper holders and liners, feminine hygiene garments such as panty liners, absorbent inserts, and the like.

"Proximal" and "Distal" refer respectively to the location of an element relatively near to or far from the longitudinal or lateral centerline of a structure (*e.g.*, the proximal edge of a longitudinally extending element is located nearer to the longitudinal centerline than the distal edge of the same element is located relative to the same longitudinal centerline).

"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (*i.e.*, element or surface is proximate to the wearer's garments that may be worn over the disposable absorbent article).

"Longitudinal" refers to a direction running substantially perpendicular from a waist edge to an opposing waist edge of the article and generally parallel to the maximum linear dimension of the article. Directions within 45 degrees of the longitudinal direction are considered to be "longitudinal"

"Lateral" refers to a direction running from a longitudinal edge to an opposing longitudinal edge of the article and generally at a right angle to the longitudinal direction. Directions within 45 degrees of the lateral direction are considered to be "lateral."

"Disposed" refers to an element being located in a particular place or position.

"Joined" refers to configurations whereby an element is directly secured to another element by affixing the element directly to the other element and to configurations whereby an element is indirectly secured to another element by affixing the element to intermediate member(s) which in turn are affixed to the other element.

"Film" refers to a sheet-like material wherein the length and width of the material far exceed the thickness of the material. Typically, films have a thickness of about 0.5 mm or less.

"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, *i.e.*, may be "vapor-permeable." As is well known in the art, a common method for measuring the permeability to water, urine, or synthetic urine of the materials typically used in absorbent articles is a hydrostatic pressure test, also called a hydrostatic head test or simply a "hydrohead" test. Suitable well known compendial methods for hydrohead testing are approved by INDA (formerly the International Nonwovens and Disposables Association, now The Association of the Nonwoven Fabrics Industry) and EDANA (European Disposables and Nonwovens Association).

The term "auxetic material" refers to materials which have a negative Poisson's Ratio, v. This means that, unlike an elastic band for example, which gets thinner when stretched, an auxetic material will thicken. Equally, if an auxetic material is compressed, it will thin. An auxetic material may comprise either a material that, due to its inherent structure has a negative Poisson's Ratio or a material that does not inherently have a negative Poisson's Ratio but has been further processed (e.g., combined with an auxetic material, bonded with a pattern causing the processed material to have auxetic behavior, formed into an auxetic macro structure, and the like) so as to be provided with such a negative ratio.

For purposes of the present invention a material or assembly of materials exhibiting "auxetic behavior" responds to an applied stress with a negative Poisson's Ratio in directions orthogonal to the applied stress.

The term "Poisson's Ratio" is defined herein as the ratio of an orthogonal strain in response to a strain resulting from a stretching force or a compressive force.

"Extensibility" and "extensible" mean that the width or length of the component in a relaxed state can be extended or increased.

"Elastic," "elastomer," and "elastomeric" refer to a material which generally is able to extend to a strain of at least 50% without breaking or rupturing, and is able to recover substantially to its original dimensions after the deforming force has been removed.

"Elastomeric material" is a material exhibiting elastic properties. Elastomeric materials may include elastomeric films, scrims, nonwovens, and other sheet-like structures.

"Outboard" and "inboard" refer respectively to the location of an element disposed relatively far from or near to the longitudinal centerline of the diaper with respect to a second element. For example, if element A is outboard of element B, then element A is farther from the longitudinal centerline than is element B.

"Pant" refers to disposable absorbent articles having a pre-formed waist and leg openings. A pant may be provided in either a permanently closed configuration or in a reclosable configuration. A pant may be donned by inserting a wearer's legs into the leg openings and sliding the pant into position about the wearer's lower torso. Pants are also commonly referred to as "closed diapers", "prefastened diapers", "pull-on diapers", "training pants" and "diaper-pants."

"Nonwoven" fabric or web means a web having a structure of individual fibers or threads that are interlaid, but not in a regular or identifiable manner as in a knitted fabric. Nonwoven fabrics or webs have been formed from many processes such as, for example, meltblowing processes, spunbonding processes, air laying processes, and bonded carded web processes. The basis weight of nonwoven fabrics is usually expressed in ounces of material per square yard (osy) or grams per square meter (gsm) and the fiber diameters are usually expressed in microns. (Note: to convert from osy to gsm, multiply osy by 33.91.)

"Woven" fabric or web means a fabric or web containing a structure of fibers, filaments, or yarns, which are arranged in an orderly, inter-engaged fashion. Woven fabrics typically contain inter-engaged fibers in a warp and fill direction. The warp direction corresponds to the length of the fabric while the fill direction corresponds to the width of the fabric. Woven fabrics can be made, for example, on a variety of looms including, but not limited to, shuttle looms, rapier looms, projectile looms, air jet looms, and water jet looms.

"Spunbonded fibers", or "spunbond fibers", means small-diameter fibers that are typically formed by extruding molten thermoplastic material as filaments from a plurality of fine capillaries of a spinneret having a circular or other configuration, with the diameter of the extruded filaments then being rapidly reduced as by, for example, in US Pat. Nos. 4,340,563, 3,692,618, 3,802,817, 3,338,992, 3,341,394, 3,502,763, 3,502,538, and 3,542,615. Spunbond fibers are quenched and generally not tacky when they are deposited onto a collecting surface. Spunbond fibers are generally continuous and often have average diameters larger than about 7 microns, and more particularly between about 10 and 30 microns. A spunbond material, layer, or substrate comprises spunbonded (or spunbond) fibers.

The term "meltblown fibers" means fibers formed by extruding a molten material, typically thermoplastic in nature, through a plurality of fine, usually circular, die capillaries as molten threads or filaments into converging high-velocity heated gas (e.g., air) streams that attenuate the filaments of molten material to reduce their diameter, which may be to microfiber diameter. Thereafter, the meltblown fibers are carried by the high-velocity gas stream and are deposited on a collecting surface to form a web of randomly dispersed meltblown fibers. Such a process is disclosed for example, in US Pat. No. 3,849,241. Meltblown fibers are microfibers which may be continuous or discontinuous, are generally smaller than 10 microns in diameter, and are generally self-bonding when deposited onto a collecting surface

The term "microfibers" means small-diameter fibers having an average diameter not greater than about 100 microns, for example, having a diameter of from about 0.5 microns to about 50 microns, more specifically microfibers may also have an average diameter of from about 1 micron to about 20 microns. Microfibers having an average diameter of about 3 microns or less are commonly referred to as ultra-fine microfibers. A description of an exemplary process of making ultra-fine microfibers may be found in, for example, US Pat. No. 5,213,881.

Absorbent Article

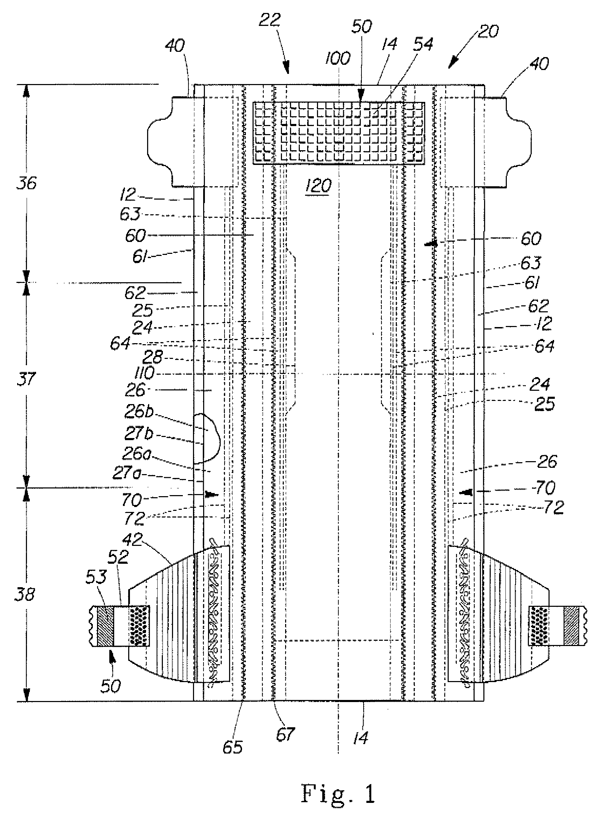

FIG. 1 is a plan view of an exemplary, non-limiting embodiment of a disposable absorbent article, diaper 20 of the present invention in a flat, uncontracted state (*i.e.*, without elastic induced contraction). FIG. 1 illustrates the various components and regions of such absorbent articles. The following discussion delineates suitable materials for use as components for such absorbent articles when the component does not comprise an auxetic material. The use of auxetic materials in such components is discussed in a subsequent section.

The garment-facing surface 120 of the diaper 20 is facing the viewer. The diaper 20 includes a longitudinal centerline 100 and a lateral centerline 110. The diaper 20 may comprise a chassis 22. The diaper 20 and chassis 22 are shown to have a front waist region 36, a rear waist region 38 opposed to the front waist region 36, and a crotch region 37 located between the front waist region 36 and the rear waist region 38. The waist regions 36 and 38 generally comprise those portions of the diaper 20 which, when worn, encircle the waist of the wearer. The waist regions 36 and 38 may include elastic elements such that they gather about the waist of the wearer to provide improved fit and containment. The crotch region 37 is that portion of the diaper 20 which, when the diaper 20 is worn, is generally positioned between the legs of the wearer.

The outer periphery of chassis 22 is defined by longitudinal side edges 12 and end edges 14. The chassis 22 may have opposing longitudinal side edges 12 that are oriented generally parallel to the longitudinal centerline 100. However, for better fit, longitudinal side edges 12 may be curved or angled to produce, for example, an "hourglass" shape diaper when viewed in a plan view. The chassis 22 may have opposing end edges 14 that are oriented generally parallel to the lateral centerline 110.

The chassis 22 may comprise a liquid permeable topsheet 24 having longitudinal side edges 25, a backsheet 26, and an absorbent core 28 between the topsheet 24 and the backsheet 26. The absorbent core 28 may have a body-facing surface and a garment facing-surface. The topsheet 24 may be joined to the core 28 and/or the backsheet 26. The backsheet 26 may be joined to the core 28 and/or the topsheet 24. It should be recognized that other structures, elements, or substrates may be positioned between the core 28 and the topsheet 24 and/or backsheet 26. In certain embodiments, the chassis 22 comprises the main structure of the diaper 20 with other features may added to form the composite diaper structure. While the topsheet 24, the backsheet 26, and the absorbent core 28 may be assembled in a variety of well-known configurations, preferred diaper configurations are described generally in US Pat. Nos. 3,860,003; 5,151,092; 5,221,274; 5,554,145; 5,569,234; 5,580,411; 5,643,239; and 6,004,306.

The topsheet 24 is generally a portion of the diaper 20 that may be positioned at least in partial contact or close proximity to a wearer. Suitable topsheets 24 may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. The topsheet 24 is generally supple, soft feeling, and non-irritating to a wearer's skin. Generally, at least a portion of the topsheet 24 is liquid pervious, permitting liquid to readily penetrate through the thickness of the topsheet 24. An exemplary topsheet 24 is available from BBA Fiberweb, Brentwood, TN as supplier code 055SLPV09U.

Any portion of the topsheet 24 may be coated with a lotion as is known in the art. Examples of suitable lotions include those described in US Pat. Nos. 5,607,760; 5,609,587; 5,635,191; and 5,643,588. The topsheet 24 may be fully or partially elasticized or may be foreshortened so as to provide a void space between the topsheet 24 and the core 28. Exemplary structures including elasticized or foreshortened topsheets are described in more detail in US Pat. Nos. 4,892,536; 4,990,147; 5,037,416; and 5,269,775 and in published US Pat. Application 2004/0162538.

The absorbent core 28 may comprise a wide variety of liquid-absorbent materials commonly used in disposable diapers and other absorbent articles. Examples of suitable absorbent materials include comminuted wood pulp, which is generally referred to as air felt creped cellulose wadding; melt blown polymers, including co-form; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials. These materials may be combined to provide a core 28 in the form of one or more layers (individual layers not shown) that may include fluid handling layers such as acquisition members, distribution members and storage members. Such absorbent cores 28 may also include layers (not shown) to stabilize other core components. Such layers include a core cover and a dusting layer. A suitable material for such layers is a spunbonded/meltblown/spunbonded nonwoven having a basis weight between about 10 and 15 $g/m^2$ (the meltblown layer comprises <5$g/m^2$) as is available from Avgol America, Inc. of Knoxville, NC. For example, Exemplary absorbent structures for use as the absorbent core 28 are described in US Pat. Nos. 4,610,678; 4,673,402; 4,834,735; 4,888,231; 5,137,537; 5,147,345; 5,342,338; 5,260,345; 5,387,207; 5,397,316; 5,625,222, and 5,643,239; in US Pat Application 09/308430, filed on November 18, 1997; and in published US Pat. Application Nos. 04/0162536 and 04/0167486.

The backsheet 26 is generally positioned such that it may be at least a portion of the garment-facing surface 120 of the diaper 20. Backsheet 26 may be designed to prevent the exudates absorbed by and contained within the diaper 20 from soiling articles that may contact the diaper 20, such as bed sheets and undergarments. In certain embodiments, the backsheet 26 is substantially water-impermeable. Backsheet 26 materials may include films such as those manufactured by Tredegar Industries Inc. of Terre Haute, IN and sold under the trade names X15306, X10962, and X10964. Other backsheet 26 materials may include breathable materials that permit vapors to escape from the diaper 20 while still preventing exudates from passing through the backsheet 26. Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, TX, under the designation EXXAIRE. Suitable breathable composite materials comprising polymer blends are available from Clopay Corporation, Cincinnati, OH under the name HYTREL blend P18-3097. Such breathable composite materials are described in greater detail in PCT Application No. WO 95/16746 and US Pat. No. 5,865,823. Other breathable backsheets including nonwoven webs and apertured formed films are described in US Pat. No. 5,571,096. An exemplary, suitable backsheet is disclosed in US Pat. No. 6,107,537. Other suitable materials and/or manufacturing techniques may be used to provide a suitable backsheet 26 including, but not limited to, surface treatments, particular film selections and processing, particular filament selections and processing, etc.

Backsheet 26 may also consist of more than one layer, as illustrated in the cut-away of FIG. 1. The backsheet 26 may comprise an outer cover 26a and an inner layer 26b. The outer cover 26a may have longitudinal side edges 27a and the inner layer 26b may have longitudinal side edges 27b. The outer cover 26a may be made of a soft, non-woven material. The inner layer 26b may be made of a substantially water-impermeable film. The outer cover 26a and an inner layer 26b may be joined together by adhesive or any other suitable material or method. A particularly suitable outer cover 26a is available from Corovin GmbH, Peine, Germany as supplier code A18AH0, and a particularly suitable inner layer 26b is available from RKW Gronau GmbH, Gronau, Germany as supplier code PGBR4WPR. While a variety of backsheet configurations are contemplated herein, it would be obvious to those skilled in the art that various other changes and modifications can be made without departing from the spirit and scope of the invention.

The diaper 20 may also include a fastening system 50. When fastened, the fastening system 50 interconnects the front waist region 36 and the rear waist region 38 resulting in a waist circumference that may encircle the wearer during wear of the diaper 20. The fastening system 50 may comprises a fastener such as tape tabs, hook and loop fastening components, interlocking fasteners such as tabs & slots, buckles, buttons, snaps, and/or hermaphroditic fastening components, although any other known fastening means are generally acceptable. Some exemplary surface fastening systems are disclosed in US Pat. Nos. 3,848,594; 4,662,875; 4,846,815; 4,894,060; 4,946,527; 5,151,092; and 5,221,274. An exemplary interlocking fastening system is disclosed in US Pat. No. 6,432,098. The fastening system 50 may also provide a means for holding the article in a disposal configuration as disclosed in US Pat. No. 4,963,140. The fastening system 50 may also include primary and secondary fastening systems, as disclosed in US Pat. No. 4,699,622. The fastening system 50 may be constructed to reduce shifting of overlapped portions or to improve fit as disclosed in US Pat. Nos. 5,242,436; 5,499,978; 5,507,736; and 5,591,152.

FIG. 1 depicts a fastening system 50 having an engaging member 52 and a receiving member 54. The engaging member 52 is shown having an engaging surface 53 that may comprise hooks, loops, an adhesive, a cohesive, or other fastening member. The receiving member 54 may have a surface that allows for engagement of the engaging member 52. The receiving member 54 may comprise hooks, loops, an adhesive, a cohesive, or other fastening component that can receive the engaging member 52. Suitable engaging member 52 and receiving member 54 combinations include but are not limited to hooks/loop, hooks/hooks, adhesive/polymeric film; cohesive/ cohesive, adhesive/adhesive; tab/slot; and button/button hole.

The diaper 20 may include barrier cuffs 60 and/or gasketing cuffs 70. Gasketing cuffs 70 may also be referred to as outer leg cuffs, leg bands, side flaps, leg cuffs, or elastic cuffs. Barrier cuffs 60 may also be referred to as second cuffs, inner leg cuffs or "stand-up" elasticized flaps.

The gasketing cuff 70 may be substantially inelastic or may be elastically extensible to dynamically fit at the wearer's leg. The gasketing cuff 70 may be formed by one or more elastic members 72 (such as elastic strands) operatively joined to the topsheet 24, backsheet 26, or any other suitable substrate used in the formation of the diaper 20. Suitable gasketing cuff construction is further described in US Pat. No. 3,860,003

The barrier cuff 60 may have a distal edge 61 and a proximal edge 63 that run substantially parallel to the longitudinal centerline 100. The barrier cuff 60 may span the entire longitudinal length of the diaper 20. The barrier cuff 60 may be formed by a flap 62 and an elastic member 64 (such as elastic strands). The flap 62 may be a continuous extension of any of the existing materials or elements that form the diaper 20. In other embodiments, such as shown in FIG. 1, the barrier cuff 60 may be a discrete element. In such embodiments, the barrier cuff 60 comprising the flap 62 and the elastic member 64 may be formed then joined to the chassis 22 by a bond 65.

The flap 62 may comprise a variety of substrates such as plastic films and woven or nonwoven webs of natural fibers (e.g., wood or cotton fibers), synthetic fibers (e.g., polyester or polypropylene fibers), or a combination of natural and synthetic fibers. In certain embodiments, the flap 62 may comprise a nonwoven web such as spunbond webs, meltblown webs, carded webs, and combinations thereof (e.g., spunbond-meltblown composites and variants). Laminates of the aforementioned substrates may also be used to form the flap 62. A particularly suitable flap may comprise a nonwoven available from BBA Fiberweb, Brentwood, TN as supplier code 30926. A particularly suitable elastic member is available from Invista, Wichita, KS as supplier code T262P. Further description of diapers having barrier cuffs and suitable construction of such barrier cuffs may be found in US Pat. Nos. 4,808,178 and 4,909,803. The elastic member 64 generally spans the longitudinal length of the barrier cuff 60. In other embodiments, the elastic member 64 may span at least the longitudinal length of the barrier cuff 60 within the crotch region 37. It is desirable that the elastic member 64 exhibits sufficient elasticity such that the proximal edge 63 of the barrier cuff 60 remains in contact with the wearer during normal wear, thereby enhancing the barrier properties of the barrier cuff 60. The elastic member 64 may be connected to the flap 62 at opposing longitudinal ends. In certain embodiments, the flap 62 may be folded over onto itself so as to encircle the elastic member 64. A bond 67 may be used to secure the folded section of the flap 62.

The barrier cuffs 60 and/or gasketing cuffs 70 may be treated, in full or in part, with a lotion, as described above with regard to topsheets, or may be fully or partially coated with a hydrophobic surface coating as detailed in U.S. Application No. 11/055,743, which was filed February 10, 2005.

In another suitable embodiment (not shown), described in US Pat. 5,643,239, a barrier material which underlies and partially encapsulates core 28 also forms a portion of barrier cuff 60.

The diaper 20 may also include a waist feature 56 which may be disposed in either of front waist region 36 or rear waist region 38 or in both waist regions. In one embodiment comprising a waist feature, the waist feature is disposed adjacent t least one end edge 14 of diaper 20. Waist feature 56 can comprise one or both of front waist feature 56a and rear waist feature 56b. In one embodiment the waist feature 56 can comprise an elasticized waistband as described in US Pats. 4,515,595 and 5,246,433. In another embodiment the waist feature 56 can comprise a bolstering waist feature as described in US Pat. 5,649,920. Waist feature 56 can also comprise a waist shield for prevention of wicking of absorbed liquids from absorbent core 28 through topsheet 24 and onto a wearer's skin as is described in US Pat. 5,019,065. Waist feature 56 can also be operatively associated with one or both of front ears 40 and back ears 42. In one embodiment waist feature 56 is disposed between topsheet 24 and backsheet 26. In another embodiment waist feature 56 is disposed on either of topsheet 24 or backsheet 26.

The diaper 20 may include front ears 40 and back ears 42. The front and/or back ears 40, 42 may be unitary elements of the diaper 20 (i.e., they are not separately manipulative elements secured to the diaper 20, but rather are formed from and are extensions of one or more of the various layers of the diaper). In certain embodiments, the front and/or back ears 40, 42 may be discrete elements that are joined to the chassis 22, as shown in FIG. 1. Discrete front and/or back ears 40, 42 may be joined to the chassis 22 by any bonding method known in the art such as adhesive bonding, pressure bonding, heat bonding, and the like. In other embodiments, the front and/or back ears 40, 42 may comprise a discrete element joined to the chassis 22 with the chassis 22 having a layer, element, or substrate that extends over the front and/or back ear 40, 42. The front ears 40 and back ears 42 may be extensible, inextensible, elastic, or inelastic. The front ears 40 and back ears 42 may be formed from nonwoven webs, woven webs, knitted fabrics, polymeric and elastomeric films, apertured films, sponges, foams, scrims, and combinations and laminates thereof. In certain embodiments the front ears 40 and back ears 42 may be formed of a nonwoven/elastomeric material laminate or a nonwoven/elastomeric material/nonwoven laminate. A suitable elastic back ear 42 may be a laminate comprising an elastomeric film (such as is available from Tredegar Corp, Richmond, VA, as supplier code X25007) disposed between two nonwoven layers (such as is available from BBA Fiberweb, Brentwood, TN as supplier code FPN332). While the following embodiments are directed to back ear 42 design and construction, these embodiments are equally applicable to front ear 40 design and construction. It should be recognized that any combination of the following embodiments may be used for the back ear 42 and/or the front ear 40.

In alternative embodiments, the diaper 20 may be preformed by the manufacturer to create a pant. A pant may be preformed by any suitable technique including, but not limited to, joining together portions of the article using refastenable and/or non-refastenable bonds (e.g., seam, weld, adhesive, cohesive bond, fastener, etc.). For example, the diaper 20 of FIG. 1 may be manufactured with the fastening system 50 engaged (*i.e.,* the engaging member 52 is joined to the receiving member 54). As an additional example, the diaper 20 of FIG. 1 may be manufactured with the front ears 40 joined to the back ears 42 by way of a bond such as an adhesive bond, a mechanical bond, or some other bonding technique known in the art. Suitable pants are disclosed in US Pat. Nos. 5,246,433; 5,569,234; 6,120,487; 6,120,489; 4,940,464; 5,092,861; 5,897,545; and 5,957,908.

Auxetic Materials and Use of Such Materials in Absorbent Articles

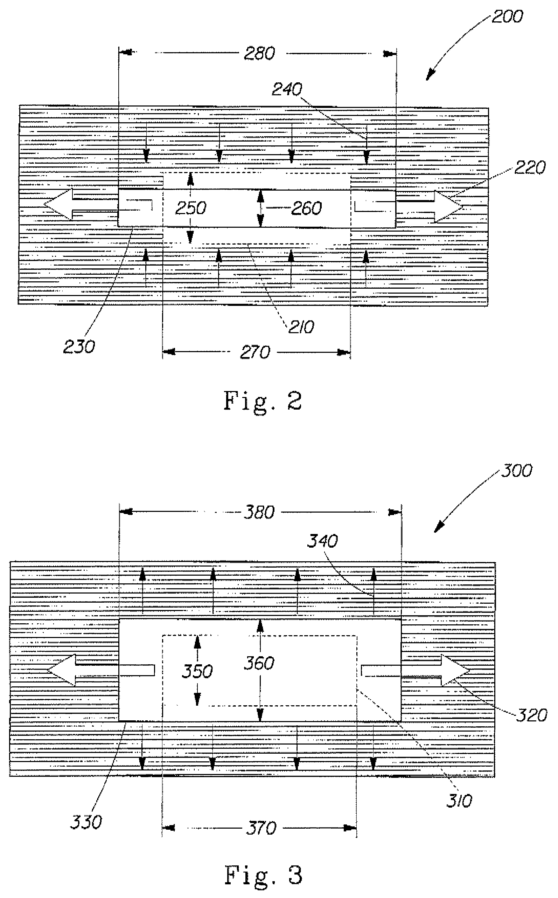

As described briefly above, an auxetic material is one which has a negative Poisson's Ratio, v. The effect of such a negative Poisson's Ratio can best be exemplified by reference to FIGs. 2 and 3 which show the effect of an applied tension on a material having a positive Poisson's Ratio (non-auxetic) and a material having a negative Poisson's Ratio (auxetic).

Referring to FIG. 2 when a sample of a non-auxetic material 200 having an original relaxed, rectangular configuration, shown as dashed lines 210, has a tensile force 220 applied thereto so as to cause it to assume a tensioned state, shown as solid lines 230. As can be seen tensile force causes the sides of material 200 that are parallel to the direction of tensile force 220 to move perpendicularly, shown as arrows 240 so as to reduce the dimension therebetween as it moves from a first dimension 250 to a second dimension 260. Similarly the distance between sides lying perpendicular to the direction of tensile force 220 moves from a first dimension 270 to a longer second dimension 280. Poisson's Ratio for such non-auxetic materials is defined as:

$$v = \frac{(250 - 260)}{(280 - 270)}$$

Referring to FIG. 3 when a sample of an auxetic material 300 having an original relaxed, rectangular configuration, shown as dashed lines 310, has a tensile force 320 applied thereto so as to cause it to assume a tensioned state, shown as solid lines 330. As can be seen tensile force causes the sides of material 300 that are parallel to the direction of tensile force 320 to move perpendicularly, shown as arrows 340 so as to increase the dimension therebetween as it moves from a first dimension 350 to a second dimension 360. Similarly the distance between sides lying perpendicular to the direction of tensile force 320 move from a first dimension 370 to a longer second dimension 380. Poisson's Ratio for such auxetic materials is defined as:

$$v = \frac{(350 - 360)}{(380 - 370)}$$

As will be recognized, Poisson's Ratio for auxetic material 300 is negative because dimension 360 is larger than dimension 350 due to the expansion thereof.

Auxetic materials are available in a wide variety of forms, including, but not limited to, polymeric foams, polymeric fibers, polymeric films and molecular level auxetics. As will be discussed below auxetic structures may be inherently auxetic or may be processed so as to be provided with auxetic properties.

Auxetic Foams

Auxetic foams may be produced by heating a standard open cell foam (e.g., a polyurethane foam) and compressing it in all three dimensions while at an elevated temperature. The foam is then cooled while under compression. Without being bound by theory, it is believed that such processing transforms the foam into one where the ribs/walls defining the foam cells are buckled inwardly forming a re-entrant cell structure. Longitudinal expansion of such re-entrant foams results in lateral expansion (i.e., such foams are an auxetic material). Auxetic materials of this type are described in US Pat. 4,668,557 and a scaled up process for making such materials is described in PCT publication WO 99/25530.

It is believed that foam materials of this type are suitable for one or more of the following absorbent article components: cuffs (both barrier cuff 60 and gasketing cuff 70); waist features 56, particularly waist bands; and cores 28, particularly acquisition and storage members. As will be recognized by a review of PCT Publication WO 99/255530 auxetic foams can be produced from polyurethane foams. As is well known, polyurethane foams can have a wide variety of properties, depending on the specific diisocyanate and glycol chosen for reaction. For example, a polyester-based prepolymer with terminal hydroxyl groups can be prepared for reaction with the diisocyanate for use as an elastomer, specific blowing agents can be used to control cell size in the foam so as to make such foams suitable for a core component and the like.

Auxetic Fabrics

Auxetic fibers and fabrics produced therefrom are also useful in constructing some embodiments of the present invention. Without being bound by theory it is believed that fabrics comprising such fibers have many desirable properties including, but not limited to, fracture toughness, energy absorption, indent resistance, impact resistance opacity under strain and breathability. Such auxetic fabrics can have particular utility for use as topsheets, the fabric component of a "cloth-like" backsheet, fabric components of a core (e.g., core covers, acquisition members, distribution members, dusting layers), cuff components and the like. Auxetic fabrics are also suitable for use in fastening system 50. For example a fastening system of the type described in US Pat. 6,432,098 can be produced by printing an auxetic pattern of a thermoplastic as described below.

In one embodiment an auxetic fabric can help prevent loss of area coverage by chassis 22 as absorbent article 20 becomes loaded with wearer exudates. As will be recognized, loading of absorbent article 20 can cause sagging thereof with resulting longitudinal stretching of topsheet 24 and backsheet 26. If one or both of topsheet 24 and backsheet 26 comprise an auxetic fabric it is believed that there will be reduced lateral contraction thereof in response to a longitudinal stretching with a resulting reduction in loss of area coverage due to lateral necking of topsheet 24 and/or backsheet 26 in response to longitudinal stresses from absorption of exudates.

Similarly, in another embodiment use of an auxetic fabric in topsheet 24 and/or backsheet 26 can enable chassis 22 to provide needed area coverage upon application while less material is used to form chassis 22 because of the expansive nature of auxetic materials when they are elongated. Said another way, stretching such materials when absorbent article 20 is applied to a wearer causes chassis 22 to expand from an original smaller configuration so as to provide needed area coverage.

In another embodiment auxetic fabrics provide desirable opacity to portions of an absorbent article that are subjected to strain. For example, front or back ears 40, 42 are stretched when absorbent article 20 is worn. Unless some provision is made (e.g., a sufficiently high basis weight for a non-auxetic nonwoven fabric comprising ears 40, 42) this stretching can cause an undesirable reduction of opacity. As will be recognized, the expansion of an auxetic fabric (or auxetic fibers comprising such a fabric) when it is stretched will also provide such desirable opacity. In some embodiments the component has an opacity ratio that is greater than 1.0 : 1.0. In other embodiments the ratio is greater than 1.05 : 1.0. A method for measuring opacity can be found in the TEST METHODS section below.

On suitable method of producing auxetic fibers is described in US Pat. 6,878,320. As described therein a polymeric powder is heated to a temperature sufficient to allow some degree of surface melting yet not high enough to enable bulk melting. Sufficient pressure is simultaneously applied (e.g., via an extruder) to cause the powder particles to adhere to each other forming a cohered structure that is then extruded in the form of fibers and/or filaments. When extended, such fibers expand or contract in a direction that is orthogonal to an applied extension or compression stress. As will be recognized such fibers when formed are suitable for formation into, for example, staple so they can be converted into a nonwoven structure or for weaving for formation of a woven fabric.

Another method to produce auxetic fibers is to produce them from an auxetic polymer. Such polymers can comprise a structure having a main polymer backbone chain with transversely attached rigid rods attached thereto. When the polymer chain is stretched the rods "stand up" providing a steric interference with alignment of the stretched polymer chains with a resulting minimization of shrinkage in a direction perpendicular to the stretching force. A further description of such molecular-level auxetic materials can be found in an online article entitled 'Textile Fibers Engineered from Molecular Auxetic Polymers"[1]

Auxetic fabrics can also be fabricated by providing a non-auxetic fabric with a bonding pattern causing it to respond to an applied stress with negative shrinkage in an

1. http://www.ptfe.gatech.edu/faculty/mcmullan/paper.php
2. http://silver.neep.wisc.edu/-lakes/Poisson.html

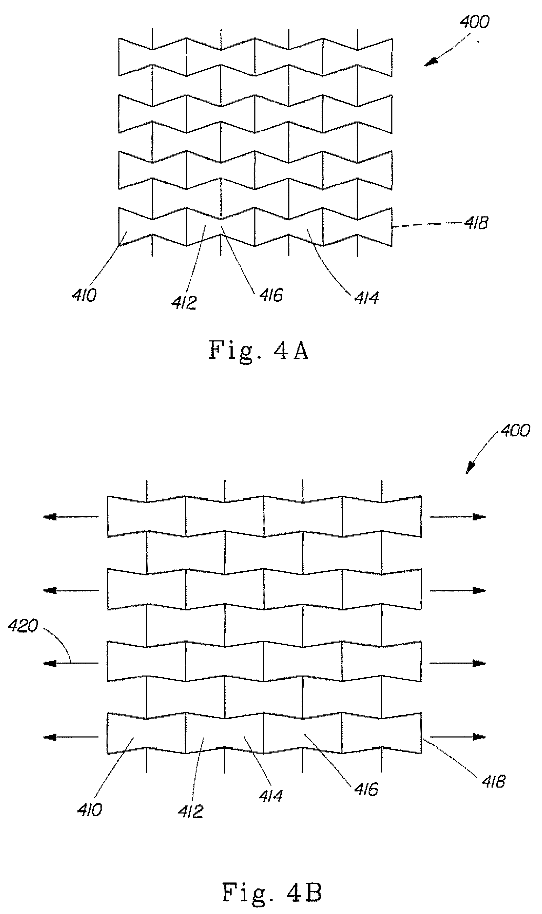

orthogonal direction thereto. Patterns of this type are described in published US Pat. Appl. 2005/0142331A1. An exemplary pattern is shown in FIGS. 4a and 4b which are adapted from Lakes, R., Negative Poisson's Ratio Materials[2]. As shown therein, pattern 400 forms a multiplicity of individual cells 410 formed from a multiplicity of struts 418. Each cell 410 includes a pair of expanded regions 412 and 414 separated by a constricted region 416. Cells 410 are shown in an unstressed state in FIG. 4a.

When cells 410 are transitioned from an unstressed state to the stressed state shown in FIG. 4b by application of a force (shown as arrows 420) struts 418 carry the force through the auxetic material causing constricted regions 416 to open up (e.g., by separation of fibers within cells 410) in a direction orthogonal to force 420 substantially preserving the dimension of the auxetic material in the direction orthogonal to force 420.

Such bonding patterns may be provided by thermal bonding of the nonwoven material, if it comprises thermoplastic fibers; application of a melted thermoplastic (e.g., a hot melt adhesive or a thermoplastic polymer) using printing techniques; or other means as may be known to the art.

Auxetic Films

Auxetic films may also be provided for use in absorbent articles according to the present invention. Such materials are particularly suitable for use as a backsheet 26 for breathability and preservation of area coverage reasons.

In one embodiment an auxetic film may be produced by embossing a film material with a pattern similar to those discussed above with respect to auxetic fabrics. For example a film can be provided with one of the patterns suitable for formation of an auxetic fabric that is comprised of regions with substantially molecular level deformation and regions with substantially geometric deformation as described in US Pat. 5,518,801

so that the regions with substantially geometric deformation act in the same manner as the struts 418 described above.

In another embodiment of an auxetic film the film can be produced by extrusion of "sintered" polymeric powder using a process similar to that described for extrusion of auxetic fibers described above and in US Pat. 6,878,320. Without being bound by theory it is believed that a film material produced in this manner would be particularly suitable for use as a component of backsheet 26 because the sintering process would provide void volume between the powder particles allowing a pathway for diffusion of water vapor making the material "breathable".

In yet another embodiment an auxetic film (or fiber) can be produced according to the methods described in US Pat. 6,743,388 which describes extrusion of polymeric pseudo-gels through an orifice, cooling the gel to precipitate the polymer, removing the solvent and stretching the remaining polymer structure using means known to the art (e.g., draws between nips and tentering) so as to provide auxetic properties.

Macro Structures

In other embodiments auxetic materials suitable for use in absorbent articles according to the present invention can be assembled by joining materials to form a macro auxetic structure. For example, a thermoplastic material could be printed using one of the patterns suitable for provision of auxetic behavior as described hereinabove forming a substantially planar auxetic structure. If desired, the first planar structure could be provided with vertical projections of varying length perpendicular to the first planar structure and layers of such a modified structure could be joined together so as to form a structure also having a multiplicity of cells similar to cells 410 in a plane perpendicular to the first planar structure that has three dimensional auxetic properties. As will be recognized the dimensions of such printed "struts" could be varied to suit a large variety of needs. A material of this type would be quite similar to the auxetic foams discussed above and would be suitable for essentially the same components of an absorbent article.

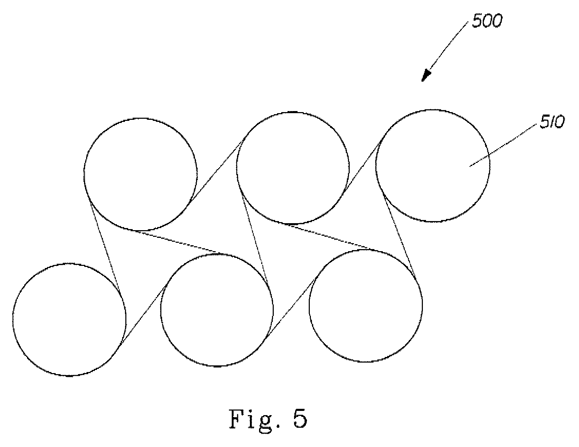

In yet another embodiment an auxetic macro structure 500 can be formed from a noncentrosymmetric assembly of macro elements 510 such as the structure shown in FIG.5. This figure is shown in and structures of this type are discussed in greater detail in Lakes, R. S., "Deformation mechanisms of negative Poisson's Ratio materials: structural aspects", J. Materials Science, 26, 2287-2292 (1991). For example such a structure can be assembled by rolling a web-like material into the structure shown for elements 510 shown in FIG. 5 and connecting the elements as shown to form auxetic structure 500.

TEST METHODS

Poisson's Ratio

This method is suitable for measuring the Poisson's Ratio for an auxetic material that is in a web form.

Apparatus

Tensile Tester: A suitable tensile tester is available from MTS Synergie 100 as is available from MTS or Eden Prairie, MN.

Load Cell: The load cell should be selected to be sure that the measured loads fall within 10-90% of the cell capacity. Suitable cells are also available from MTS.

Data Acquisition and Analysis Software: Software compatible with tensile tester used. TestWorks 4 available from MTS is suitable for the Synergie series.

Laser Scan Micrometer: Suitable for measuring dimensions of ±0.1 mm. A suitable instrument is available from Keyence Corporation of Osaka, Japan as the model LS-5121.

Sample Cutter: Suitable for cutting desired sample dimensions (25 mm X 20 cm) as is available from Thwing-Albert Instrument Company of Philadelphia, PA as the JDC Precision Sample Cutter.

Sample Preparation

1. Open any container and remove individual absorbent articles therefrom. Remove a sufficient number of articles to provide material for subsequent evaluation.

2. Condition the individual absorbent articles at 22±22°C/50±2% Relative Humidity for 24 hours prior to testing.

3. Where the material to be tested is obtained from an absorbent article, each article should be disassembled so as to provide the component of interest (e.g., a waist feature or a cuff) in a manner that minimally disturbs the structure of any layers comprising the absorbent article. For example, adhesively joined layers can be separated by first freezing them using a freeze spray such as Freeze-It® as is available from ITW Chemtronics Americas of Kennesaw, GA.

4. Cut the absorbent article component being evaluated to a width of 25 mm using the sample cutter. Preserve as much of the length as possible consistent with having an isotropic composition. If possible, cut the sample to a length of 20 cm. If dimensions of the isotropic region of the material are less than either 25 mm wide or 20 cm long, measure the dimensions of the largest sample of isotropic material that can be obtained. As will be recognized, if sample size results in a gage length of less than 10 cm (see below) equipment setup modifications must be made to accommodate the reduced sample size.

Operation All tests are conducted at 22 ± 2°C and 50 ± 2% relative humidity unless specified otherwise.

1. Set up and calibrate the tensile tester for tensile operation at a gage length (jaw separation) of 10 cm as described in the manufacturer's operating instructions. Also, set up and calibrate the laser caliper gage according to the manufacturer's instructions.

2. Place one end of the sample in the upper jaw of the tensile tester taking care to avoid wrinkles provides a vertical orientation.

3. Adjust the sample so there is no slack therein, applying no more than 0.05N of tension and place the other end of the sample in the lower jaw.

4. While the sample is being held in the tensile tester measure and record jaw separation ($l_{li}$) from the tensile tester display. Record sample width ($w_{li}$) and sample thickness ($t_{li}$) using the laser caliper gage. Insure that the light beam from the gage is perpendicular to the surface which is being measured for width or caliper. Record $l_{li}$, $w_{li}$ and $t_{li}$ where i is the sample number.

5. After all caliper measurements are completed, initiate a tensile cycle and extend the sample to 25% elongation.

6. While the sample is being held in the tensile tester measure and record jaw separation ($l_{2i}$) the tensile tester display. Record sample width ($w_{2i}$) and sample thickness ($t_{2i}$) using the laser caliper gage. Insure that the light beam from the gage is perpendicular to the surface which is being measured for width or caliper. Record $l_{2i}$, $w_{2i}$ and $t_{2i}$ where i is the sample number.

7. Repeat steps 2-6 for four more samples.

Calculation

Poisson's Ratio

Poisson's Ratio for each sample i is calculated according to the following equations

Width:

$$\nu_{wi} = (w_{2i} - w_{1i})/ (l_{2i} - l_{1i})$$

Thickness:

$$\nu_{ti} = (t_{2i} - t_{1i})/(l_{2i} - l_{1i})$$

Report the average value for each sample set as $\nu_w$ and $\nu_t$.

Opacity

This method is suitable for measurement of the opacity and opacity ratio of absorbent article components.

Apparatus

Reflectance Spectrophotometer The Hunter Labscan XE available from HunterLab Associates, Inc., Reston, VA or equivalent reflectance spectrophotometer with 45°/0° geometry optics is suitable.

Data Acquisition and Analysis Software Software compatible with the reflectance spectrophotometer of choice should be used. Universal Software from HunterLab is suitable for use with the Labscan XE instrument.

Tension Grips Suitable for holding a sample in a tensioned condition at 25% elongation without wrinkles in the sample.

Sample Preparation

1. Open any container and remove individual absorbent articles therefrom. Remove a sufficient number of articles to provide material for subsequent evaluation.

2. Condition the individual absorbent articles at $22\pm22°C/50\pm2\%$ Relative Humidity for 24 hours prior to testing.

3. Where the material to be tested is obtained from an absorbent article, each article should be disassembled so as to provide the component of interest (e.g., a waist feature or a cuff) in a manner that minimally disturbs the structure of any layers comprising the absorbent article. For example, adhesively joined layers can be separated by first freezing them using a freeze spray such as Freeze-It® as is available from ITW Chemtronics Americas of Kennesaw, GA.

4. Cut the absorbent article component being evaluated to a size that at least covers the aperture in the reflectance spectrophotometer with sufficient additional sample for the reference marks of step 5 and area for the grips that hold the sample under tension. If possible a sample size that is approximately 4" square (10 cm square) should be used. Scissors or other suitable device may be used. The sample should be free of dirt and other contamination.

5. Mark the sample with a pair of reference marks that are perpendicular to the direction of expected strain when the absorbent article is worn. The reference marks should be slightly wider than the aperture of the reflectance spectrophotometer.

<u>Operation</u> All tests are conducted at 22 ± 2°C and 50 ± 2% relative humidity unless specified otherwise.

1. Before testing is begun, the spectrophotometer is calibrated using the black and white standard tiles supplied with the instrument, according to the manufacturer's instructions or other accepted standards of practice. Measurements are made using a 2" (50 mm) port with the color scale set to XYZ, the Observer set to 10°, and the Illuminant set to D65.

2. Place the sample on top of the standard white tile. Load the sample onto the spectrophotometer such that the sample/white tile completely covers the instrument port. Read and record the "Y' value from the spectrophotometer to the nearest 0.1 unit as $Y_{UWi}$ where I is the replicate number.

3. Remove the sample and place the same sample on top of the standard black tile. Again, load the sample onto the spectrophotometer such that the sample/black tile completely covers the instrument port. Read and record the "Y' value from the spectrophotometer to the nearest 0.1 unit as $Y_{UBi}$.

4. Remove the sample and secure one side of the sample in the tension grips. Slowly elongate the sample so the distance between the reference marks has increased by 25% and secure the other end of the sample in the tension grips. The sample should be positioned in the tension grips and elongated so that the direction of the applied tension is parallel to the direction of tension when the sample is part of an absorbent article that is being worn.

5. Place the tensioned sample on top of the standard white tile. Load the sample onto the spectrophotometer such that the sample/white tile completely covers the instrument port. Read and record the "Y' value from the spectrophotometer to the nearest 0.1 unit as $Y_{TWi}$.

6. Remove the tensioned sample and place the same sample on top of the standard black tile. Again, load the sample onto the spectrophotometer such that the sample/black tile completely covers the instrument port. Read and record the "Y' value from the spectrophotometer to the nearest 0.1 unit as $Y_{TBi}$.

7. Repeat steps 2-6 four more times.

<u>Calculation and Reporting</u>

Opacity is reported as a percentage and is calculated by dividing the reflectance obtained from the sample with the black tile, by the reflectance obtained from the sample with the white tile, multiplied by 100, and reported to 0.1%. The equation is as follows:

$$\text{Percent Opacity (UorT)}_i = [\ (Y_{B(U \text{ or } T)i}) \div (Y_{W(U \text{or} T)i})\ ] \times 100$$

Determine $Y_{Ui}$ and $Y_{Ti}$ for each replicate. Report the average of $Y_{Ui}$ as the untensioned opacity $Y_U$ and the average of $Y_{Ti}$ as the tensioned opacity $Y_T$. The opacity ratio is defined as

$$\text{Opacity Ratio} = Y_T/Y_U$$

The dimensions and values disclosed herein are not to be understood as being strictly limited o the exact numerical values recited, Instead, unless otherwise specified, each such dimension is intended to mean both the recited value and a functionally equivalent range surround that value. For example, a dimension disclosed as "40 mm" is intended to mean "about 40 mm".

**Claims**

1.   A disposable absorbent article comprising at least one component comprising an auxetic material, the article having a longitudinal centerline and a lateral centerline, a front waist region with a front waist edge, a rear waist region with a rear waist edge, a crotch region disposed between said front and rear waist regions, and two spaced apart longitudinal side edges joining said front waist edge to said rear waist edge, wherein the component is a waist feature.

2. The disposable absorbent article of Claim 1 comprising a topsheet, a backsheet, and an absorbent core disposed between the topsheet and the backsheet.

3. The disposable absorbent article of any preceding claim wherein the auxetic material comprises auxetic fibers or auxetic foam.

4. The disposable absorbent article of any preceding claim wherein the component comprises a laminate comprising the auxetic material.

5. The disposable absorbent article of any preceding claim wherein the auxetic material causes the component to be more resistant to bending when under tension than when relaxed.

6. The disposable absorbent article of any preceding claim wherein the auxetic material causes the component to have a greater opacity when under tension than when relaxed.

**Patentansprüche**

1. Einweg-Absorptionsartikel, umfassend wenigstens eine Komponente umfassend ein auxetisches Material, wobei der Artikel eine Längsachse und eine Querachse, einen vorderen Mittelteil mit einem vorderen Taillenrand, einen hinteren Mittelteil mit einem hinteren Taillenrand, einen zwischen den vorderen und hinteren Mittelteilen angeordneten Schrittteil und zwei voneinander beabstandete Längsseitenränder, die den vorderen Taillenrand mit dem hinteren Taillenrand verbinden, aufweist, wobei die Komponente ein Taillenmerkmal ist.

2. Einweg-Absorptionsartikel nach Anspruch 1, umfassend eine Oberschicht, eine Unterschicht und einen Absorptionskern, der zwischen der Oberschicht und der Unterschicht angeordnet ist.

3. Einweg-Absorptionsartikel nach einem vorstehenden Anspruch, wobei das auxetische Material auxetische Fasern oder auxetischen Schaumstoff umfasst.

4. Einweg-Absorptionsartikel nach einem vorstehenden Anspruch, wobei die Komponente ein Laminat umfasst, das das auxetische Material umfasst.

5. Einweg-Absorptionsartikel nach einem vorstehenden Anspruch, wobei das auxetische Material bewirkt, dass die Komponente, wenn sie unter Spannung steht, widerstandsfähiger gegen Biegen ist, als wenn sie entspannt ist.

6. Einweg-Absorptionsartikel nach einem vorstehenden Anspruch, wobei das auxetische Material bewirkt, dass die Komponente, wenn sie unter Spannung steht, eine stärkere Trübung aufweist, als wenn sie entspannt ist.

**Revendications**

1. Article absorbant jetable comprenant au moins un composant comprenant un matériau auxétique, l'article possédant une ligne médiane longitudinale et une ligne médiane latérale, une région de ceinture avant avec un bord de ceinture avant, une région de ceinture arrière avec un bord de ceinture arrière, une région d'entrejambe disposée entre lesdites régions de ceinture avant et arrière, et deux bords latéraux longitudinaux espacés joignant ledit bord de ceinture avant audit bord de ceinture arrière, dans lequel le composant est un élément de ceinture.

2. Article absorbant jetable selon la revendication 1, comprenant une feuille de dessus, une feuille de fond et une âme absorbante disposée entre la feuille de dessus et la feuille de fond.

3. Article absorbant jetable selon l'une quelconque des revendications précédentes, dans lequel le matériau auxétique comprend des fibres auxétiques ou une mousse auxétique.

4.  Article absorbant jetable selon l'une quelconque des revendications précédentes, dans lequel le composant comprend un stratifié comprenant le matériau auxétique.

5.  Article absorbant jetable selon l'une quelconque des revendications précédentes, dans lequel le matériau auxétique fait en sorte que le composant soit plus résistant au pliage lorsqu'il est sous tension que lorsqu'il est relâché.

6.  Article absorbant jetable selon l'une quelconque des revendications précédentes, dans lequel le matériau auxétique fait en sorte que le composant ait une plus grande opacité lorsqu'il est sous tension que lorsqu'il est relâché.

Fig. 1

Fig. 2

Fig. 3

Fig. 4A

Fig. 4B

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6432098 B
- US 20030233082 A1
- US 3083737 A
- US 5019065 A
- US 5058247 A
- US 4688557 A
- US 6878320 B
- WO 99255530 A
- US 2005014233 A1
- US 6120487 A
- US 4340563 A
- US 3692618 A
- US 3802817 A
- US 3338992 A
- US 3341394 A
- US 3502763 A
- US 3502538 A
- US 3542615 A
- US 3849241 A
- US 5213881 A
- US 3860003 A
- US 5151092 A
- US 5221274 A
- US 5554145 A
- US 5569234 A
- US 5580411 A
- US 5643239 A
- US 6004306 A
- US 5607760 A
- US 5609587 A
- US 5635191 A
- US 5643588 A
- US 4892536 A
- US 4990147 A
- US 5037416 A
- US 5269775 A
- US 20040162538 A
- US 4610678 A
- US 4673402 A
- US 4834735 A
- US 4888231 A
- US 5137537 A
- US 5147345 A
- US 5342338 A
- US 5260345 A
- US 5387207 A
- US 5397316 A
- US 5625222 A
- US 09308430 B
- US 040162536 B
- US 040167486 B
- WO 9516746 A
- US 5865823 A
- US 5571096 A
- US 6107537 A
- US 3848594 A
- US 4662875 A
- US 4846815 A
- US 4894060 A
- US 4946527 A
- US 4963140 A
- US 4699622 A
- US 5242436 A
- US 5499978 A
- US 5507736 A
- US 5591152 A
- US 4808178 A
- US 4909803 A
- US 05574305 A
- US 5649920 A
- US 5246433 A
- US 6120489 A
- US 4940464 A
- US 5092861 A
- US 5897545 A
- US 5957908 A
- US 4668557 A
- WO 9925530 A
- US 20050142331 A1
- US 5518801 A
- US 6743388 B

**Non-patent literature cited in the description**

- **LAKES, R. S.** Deformation mechanisms of negative Poisson's Ratio materials: structural aspects. *J. Materials Science,* 1991, vol. 26, 2287-2292